# EUROPEAN PATENT APPLICATION

(11) **EP 4 491 644 A1**
(43) Date of publication of application: **15.01.2025**
(21) Application number: 23766785.2
(22) Date of filing: 06.03.2023
(51) Int. Cl.: C08F 220/06, A61K 8/81, A61K 47/32, C09K 3/00

(54) **(METH)ACRYLIC ACID/(METH)ACRYLIC ACID ALKYL ESTER CROSSLINKED COPOLYMER AND USE THEREOF**

(30) Priority: 07.03.2022 JP 2022034082
(71) Applicant: Sumitomo Seika Chemicals Co., Ltd., Kako-gun, Hyogo 675-0145 (JP)
(72) Inventor: CHUGO, Shohei, Himeji-shi, Hyogo 672-8076 (JP); NOGUCHI, Mari, Himeji-shi, Hyogo 672-8076 (JP)
(74) Representative: Vossius & Partner Patentanwälte Rechtsanwälte mbB
(86) International application number: PCT/JP2023/008304
(87) International publication number: WO 2023/171609

(57) **Abstract**

Provided is a crosslinked polymer useful as a thickener capable of preparing a viscous composition having sufficiently satisfactory transparency and spreadability when applied. More specifically, provided is a (meth)acrylic acid/(meth)acrylic acid alkyl ester crosslinked copolymer obtained by polymerizing (meth)acrylic acid, a (meth)acrylic acid alkyl ester wherein the alkyl has 18 to 24 carbon atoms, and a compound with two or more ethylenically unsaturated groups, wherein a 0.5 mass% neutralized aqueous solution of the crosslinked copolymer at 25°C has a yield stress of 20 Pa or more, and a 0.5 mass% neutralized aqueous solution of the crosslinked copolymer at 25°C has a storage elastic modulus (G') of 100 to 400 Pa at an angular frequency of 0.1 rad/s.

## Description

### Technical Field

The present disclosure relates to, for example, a specific (meth)acrylic acid/(meth)acrylic acid alkyl ester crosslinked copolymer and use of the crosslinked copolymer.

### Background Art

In the preparation of a viscous composition used for hair-styling agents, such as hair gel and hair lotion, a gelling agent is typically used to adjust the viscosity of the composition, in addition to the use of resin for hair styling, as well as water and alcohol as the dispersion medium.

The product value of such a viscous composition is significantly affected by its properties, such as a high degree of transparency and usability, including a high degree of spreadability when applied. Given this, a gelling agent that achieves excellent transparency and usability when contained in the viscous composition is highly in demand.

### Citation List

### Patent Literature

PTL 1: JP2003-012453A
PTL 2: JP2004-505902A
PTL 3: JP2014-508791A

### Summary of Invention

### Technical Problem

For example, PTL 1 discloses that a combined use of pectin, a salt that forms polyvalent cations, and a lower alcohol and/or polyhydric alcohol can produce a gel-like cosmetic product that provides a moisturizing and refreshing sensation.

PTL 2 discloses that a hair gel composition comprising a water-soluble polyalkylene glycol, a film-forming polymer, and a liquid carrier can provide good hair look and feel.

PTL 3 discloses that the use of a cosmetic composition comprising a solvent, a polyurethane urea, and a copolymer can fix hair securely while retaining elasticity.

However, conventional techniques as described above have not enabled the production of a viscous composition having sufficiently satisfactory transparency and spreadability when applied. Therefore, a viscous composition having sufficiently satisfactory transparency and spreadability when applied is still in demand.

### Solution to Problem

The present inventors have found that a (meth)acrylic acid/(meth)acrylic acid alkyl ester crosslinked copolymer obtained by polymerizing (meth)acrylic acid, a (meth)acrylic acid alkyl ester wherein the alkyl has 18 to 24 carbon atoms, and a compound with two or more ethylenically unsaturated groups, wherein a 0.5 mass% neutralized aqueous solution of the crosslinked copolymer at 25°C has a yield stress of 20 Pa or more and wherein a 0.5 mass% neutralized aqueous solution of the crosslinked copolymer at 25°C has a storage elastic modulus (G') of 100 to 400 Pa at an angular frequency of 0.1 rad/s, is capable of imparting appropriate viscosity to a liquid composition, and that the composition with imparted viscosity also has excellent transparency and usability. The present inventors then conducted further studies.

The present disclosure encompasses, for example, the subject matter described in the following Items.
Item 1. A (meth)acrylic acid/(meth)acrylic acid alkyl ester crosslinked copolymer obtained by polymerizing
   (meth)acrylic acid,
   a (meth)acrylic acid alkyl ester wherein the alkyl has 18 to 24 carbon atoms, and
   a compound with two or more ethylenically unsaturated groups,
      wherein
   a 0.5 mass% neutralized aqueous solution of the crosslinked copolymer at 25°C has a yield stress of 20 Pa or more, and
   a 0.5 mass% neutralized aqueous solution of the crosslinked copolymer at 25°C has a storage elastic modulus (G') of 100 to 400 Pa at an angular frequency of 0.1 rad/s.
Item 2. The crosslinked copolymer according to Item 1,
   wherein k is 1.2 to 6.0 wherein k represents an interaction coefficient as determined by measuring a neutralized aqueous solution of the crosslinked copolymer having an initial concentration of 0.015 mass% with an Ubbelohde dilution viscometer.
Item 3. The crosslinked copolymer according to Item 1 or 2,
   wherein the (meth)acrylic acid alkyl ester wherein the alkyl has 18 to 24 carbon atoms is
   at least one member selected from the group consisting of eicosanyl (meth)acrylate, behenyl (meth)acrylate, and tetracosanyl (meth)acrylate, or
   a mixture of two to four members selected from the group consisting of stearyl (meth)acrylate, eicosanyl (meth)acrylate, behenyl (meth)acrylate, and tetracosanyl (meth)acrylate.
Item 4. The crosslinked copolymer according to any one of Items 1 to 3,
   wherein the compound with two or more ethylenically unsaturated groups is at least one member selected from the group consisting of sucrose allyl ether and pentaerythritol allyl ether.
Item 5. The crosslinked copolymer according to any one of Items 1 to 4, which is a polymer comprising 0.5 to 3 parts by mass of the (meth)acrylic acid alkyl ester wherein the alkyl has 18 to 24 carbon atoms per 100 parts by mass of the (meth)acrylic acid.
Item 6. The crosslinked copolymer according to any one of Items 1 to 5, which is a polymer comprising 0.15 to 2 parts by mass of the compound with two or more ethylenically unsaturated groups per 100 parts by mass of the (meth)acrylic acid.
Item 7. A thickener comprising the crosslinked copolymer of any one of Items 1 to 6.
Item 8. An external composition comprising the crosslinked copolymer of any one of Items 1 to 6.
Item 9. A method for producing a (meth)acrylic
   acid/(meth)acrylic acid alkyl ester crosslinked copolymer crosslinked by a compound with two or more ethylenically unsaturated groups,
   the method comprising
   reacting (meth)acrylic acid, a (meth)acrylic acid alkyl ester wherein the alkyl has 18 to 24 carbon atoms, and a compound with two or more ethylenically unsaturated groups in an inert solvent in the presence of a radical polymerization catalyst,
   wherein the radical polymerization catalyst is an organic peroxide and is present in an amount of 0.1 to 1 part by mass per 100 parts by mass of the (meth)acrylic acid.

### Advantageous Effects of Invention

Provided is a crosslinked polymer that is capable of imparting appropriate viscosity to a liquid composition. The composition with imparted viscosity has excellent transparency and usability. Such a crosslinked polymer is particularly useful, for example, for the preparation of an external composition (an external pharmaceutical composition or cosmetic composition).

### Description of Embodiments

Embodiments encompassed by the present disclosure are described in more detail below. The present disclosure preferably encompasses, for example, a specific crosslinked polymer, a production method for the crosslinked polymer, use of the crosslinked polymer, and a viscous composition obtained by using the crosslinked polymer. However, the disclosure is not limited to these and encompasses all the matter disclosed in the present specification and recognizable to those skilled in the art.

The specific crosslinked polymer encompassed by the present disclosure is a (meth)acrylic acid/(meth)acrylic acid alkyl ester crosslinked copolymer obtained by polymerizing (meth)acrylic acid, a (meth)acrylic acid alkyl ester wherein the alkyl has 18 to 24 carbon atoms, and a compound with two or more ethylenically unsaturated groups, wherein a 0.5 mass% neutralized aqueous solution of the crosslinked copolymer at 25°C has a yield stress of 20 Pa or more, and wherein the same solution has a storage elastic modulus (G') of 100 to 400 Pa at an angular frequency of 0.1 rad/s. This crosslinked polymer is sometimes referred to as "the crosslinked polymer of the present disclosure." In the synthesis of the copolymer of the present disclosure, the compound with two or more ethylenically unsaturated groups functions as a crosslinking agent. That is, the (meth)acrylic acid/(meth)acrylic acid alkyl ester crosslinked copolymer is construed as having a structure of a polymer of (meth)acrylic acid and a (meth)acrylic acid alkyl ester crosslinked by a compound with two or more ethylenically unsaturated groups.

The crosslinked polymer of the present disclosure is obtained by polymerizing (I) (meth)acrylic acid, (II) a (meth)acrylic acid alkyl ester wherein the alkyl has 18 to 24 carbon atoms, and (III) a compound with two or more ethylenically unsaturated groups, as described above. In the present specification, these components are sometimes referred to as "the components (I) to (III)."

In the present specification, the term "(meth)acrylic" means acrylic and/or methacrylic. For the (meth)acrylic acid, either acrylic acid or methacrylic acid may be used alone or in combination.

(II) The (meth)acrylic acid alkyl ester wherein the alkyl has 18 to 24 (18, 19, 20, 21, 22, 23, or 24) carbon atoms refers to an ester of (meth)acrylic acid and a higher alcohol wherein the alkyl group has 18 to 24 carbon atoms. Examples of the (meth)acrylic acid alkyl ester include, but are not particularly limited to, esters of (meth)acrylic acid with stearyl alcohol, esters of (meth)acrylic acid with eicosanol, esters of (meth)acrylic acid with behenyl alcohol, and esters of (meth)acrylic acid with tetracosanol. Among these (meth)acrylic acid alkyl esters, stearyl methacrylate, eicosanyl methacrylate, behenyl methacrylate, and tetracosanyl methacrylate are preferable. These (meth)acrylic acid alkyl esters may be used alone or in a combination of two or more. In particular, it is more preferable to use at least one member selected from the group consisting of eicosanyl (meth)acrylate, behenyl (meth)acrylate, and tetracosanyl (meth)acrylate, or a mixture of two to four members selected from the group consisting of stearyl (meth)acrylate, eicosanyl (meth)acrylate, behenyl (meth)acrylate, and tetracosanyl (meth)acrylate. For these (meth)acrylic acid alkyl esters, for example, commercial products, such as Blemmer VMA-70 and Blemmer SMA, trade name, produced by NOF Corporation, may also be used.

Examples of (III) the compound with two or more ethylenically unsaturated groups include, but are not particularly limited to, a compound in which a polyol, such as ethylene glycol, propylene glycol, polyoxyethylene glycol, polyoxypropylene glycol, glycerol, polyglycerol, trimethylolpropane, pentaerythritol, saccharose, or sorbitol, is substituted with two or more acrylic acid esters; a compound in which a polyol mentioned above is substituted with two or more allyl ethers; and diallyl phthalate, triallyl phosphate, allyl methacrylate, tetraallyloxyethane, triallyl cyanurate, divinyl adipate, vinyl crotonate, 1,5-hexadiene, divinyl benzene, and polyallylsaccharose. Among these, the compound with two or more ethylenically unsaturated groups is preferably sucrose allyl ether, pentaerythritol allyl ether (more preferably pentaerythritol triallyl ether or pentaerythritol tetraallyl ether), tetraallyloxyethane, triallyl phosphate, or polyallylsaccharose, and more preferably sucrose allyl ether or pentaerythritol allyl ether. These compounds with two or more ethylenically unsaturated groups may be used alone or in a combination of two or more.

The crosslinked polymer of the present disclosure is preferably a polymer comprising 0.5 to 3 parts by mass of the component (II) per 100 parts by mass of the component (I). The upper or lower limit of this range (0.5 to 3) may be, for example, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, or 2.9. For example, the range may be 0.6 to 2.9.

Further, the crosslinked polymer of the present disclosure is preferably a polymer comprising 0.15 to 2 parts by mass of the component (III) per 100 parts by mass of the component (I). The upper or lower limit of this range (0.15 to 2) may be, for example, 0.2, 0.3, 0.4, 0.5, 0.6, 0.7, 0.8, 0.9, 1, 1.1, 1.2, 1.3, 1.4, 1.5, 1.6, 1.7, 1.8, or 1.9. For example, the range may be 0.2 to 1.9.

Additionally, a 0.5 mass% neutralized aqueous solution of the crosslinked polymer of the present disclosure at 25°C has a yield stress (yield value) of 20 Pa or more, as described above. This feature of the crosslinked polymer of the present disclosure is sometimes referred to as a "feature (α)." The yield stress (yield value) is preferably 20 to 200 Pa. The upper or lower limit of this preferable range may be, for example, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, 100, 110, 120, 130, 140, 150, 160, 170, 180, or 190 Pa. For example, the range may be 21 to 150 Pa.

The yield stress is measured with a rheometer. For example, the yield stress is measured by using a dynamic viscoelasticity analyzer (AR-2000ex from TA Instruments Japan) with a 60 mm (diameter), 4-degree cone. More specifically, the steady-state flow viscosity at a shear stress of 1.0 × 10⁻⁵ to 1.0 × 10³ (Pa) is measured under steady-state conditions that satisfy the following items: percentage tolerance 1.0, consecutive within tolerance 3, and maximum point time 10 min. The point at which the strain changes abruptly with respect to shear stress is determined to be the yield value (yield stress, Pa).

The neutralized aqueous solution of the crosslinked polymer used for the measurement is a 0.5 mass% neutralized aqueous solution of the polymer, as described above. For example, the neutralized aqueous solution is prepared as follows. 288.8 g of ion-exchanged water is placed in a 500 mL beaker, and 1.5 g of a crosslinked polymer is gradually added with stirring at 1500 rpm with a dispersion mixer. After subsequent stirring for 30 minutes, the dispersion mixer is turned off, and 9.7 g of a 6 mass% aqueous sodium hydroxide solution is added and stirred with a handheld mixer for 30 seconds to obtain a 0.5 mass% neutralized aqueous solution of the crosslinked polymer. An aqueous solution with a pH of 7 (measured with a pH meter at 25°C) is designated as a neutralized aqueous solution. A pH adjuster can be used for neutralization; for example, sodium hydroxide is suitable for the pH adjuster. The same applies below.

Furthermore, a 0.5 mass% neutralized aqueous solution of the crosslinked polymer of the present disclosure at 25°C has a storage elastic modulus (G') of 100 to 400 Pa at an angular frequency of 0.1 rad/s, as described above. This feature of the crosslinked polymer of the present disclosure is sometimes referred to as a "feature (β)." The upper or lower limit of this range of the storage elastic modulus (G') may be, for example, 110, 120, 130, 140, 150, 160, 170, 180, 190, 200, 210, 220, 230, 240, 250, 260, 270, 280, 290, 300, 310, 320, 330, 340, 350, 360, 370, 380, or 390 Pa. For example, the range may be 110 to 390 Pa.

The G' value (storage elastic modulus) at an angular frequency of 0.1 rad/s is a value measured with a rheometer (e.g., TA Instruments, model number: AR 2000ex) under the following conditions: measurement temperature: 25°C, angular frequency: 0.1 to 300 rad/s, and strain: 1%.

The crosslinked polymer of the present disclosure is preferably a crosslinked polymer having an interaction coefficient (k) of 1.2 and 6.0 as determined by measuring a neutralized aqueous solution of the crosslinked polymer with an Ubbelohde dilution viscometer. More specifically, the crosslinked polymer of the present disclosure is preferably a crosslinked polymer having an interaction coefficient (k) of 1.2 and 6.0 as determined by measuring a neutralized aqueous solution of the crosslinked polymer having an initial concentration of 0.015 mass% with an Ubbelohde dilution viscometer. The upper or lower limit of this range may be, for example, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, or 5.9. The range may be, for example, 1.2 to 3.

Intrinsic viscosity [η] and interaction coefficient k of the crosslinked polymer solution are more specifically determined as follows. Specifically, flow time (t) of the solution and flow time (t₀) of the solvent alone are measured with an Ubbelohde dilution viscometer in accordance with JIS K-7367-1, and ηₛₚ is determined according to the following relational expression. ηₛₚ is a specific viscosity (indicating the rate of increase in viscosity caused by a solute in a solvent). *η*ₛₚ = (*η*-*η*₀)/*η*₀ = (*ρ*t-*ρ*₀t₀)/*ρ*₀t₀ ≈ (t-t₀)/t₀ (*ρ*₀: solvent viscosity, t₀: solvent flow time, ρ: solution viscosity, t: solution flow time)

While the concentration is changed by dilution, measurement is performed at four or more points (e.g., 4, 5, or 6 points), and the results are plotted with concentration (c) on the horizontal axis and (ηₛₚ/c) on the vertical axis. Martin's equation ηₛₚ/c = [η]e^{k[η]c} with exponential approximation gives [η] and k. (Plotting the measured values gives exponential approximation in the Martin's equation. The intercept of the extrapolation of zero concentration is intrinsic viscosity [η].) Unless otherwise noted in the present specification, the unit for intrinsic viscosity [η] is dl/g.

The intrinsic viscosity [η] determined by measuring a neutralized aqueous solution of the crosslinked polymer of the present disclosure with an Ubbelohde dilution viscometer is preferably about 30 to 60. The upper or lower limit of this range may be, for example, 31, 32, 33, 34, 35, 36, 37, 38, 39, 40, 41, 42, 43, 44, 45, 46, 47, 48, 49, 50, 51, 52, 53, 54, 55, 56, 57, 58, or 59. For example, the range may be 35 to 55.

Product k[η] of intrinsic viscosity [η] and interaction coefficient k is preferably about 40 to 120. The upper or lower limit of this range may be, for example, 45, 50, 55, 60, 65, 70, 75, 80, 85, 90, 95, 100, 105, 110, or 115. For example, the range may be 45 to 115.

The crosslinked polymer of the present disclosure can be obtained, for example, by reacting (meth)acrylic acid, a (meth)acrylic acid alkyl ester wherein the alkyl has 18 to 24 carbon atoms, and a compound with two or more ethylenically unsaturated groups (i.e., the components (I) to (III)) in an inert solvent in the presence of a radical polymerization catalyst.

The amount of the (meth)acrylic acid is preferably, for example, about 6 to 25 parts by volume, more preferably 8 to 20 parts by volume, and even more preferably 8 to 15 parts by volume, per 100 parts by volume of the inert solvent.

The radical polymerization catalyst is not particularly limited as long as the crosslinked polymer of the present disclosure having the features (α) and (β) described above can be obtained, and is preferably an organic peroxide. This is because it is not easy to obtain a crosslinked polymer having both the features (α) and (β) described above without using an organic peroxide as the radical polymerization catalyst. Examples of preferable radical polymerization catalysts include benzoyl peroxide, lauroyl peroxide, cumene hydroperoxide, and tertiary butyl hydroperoxide, which are organic peroxides. The radical polymerization catalysts may be used alone or in a combination of two or more. The amount of the radical polymerization catalyst is preferably about 0.1 to 1 parts by mass per 100 parts by mass of the component (I). The upper or lower limit of this range may be, for example, 0.6, 0.7, 0.8, or 0.9 parts by mass. For example, the range may be 0.2 to 0.8 parts by mass.

In reacting the components (I) to (III) in an inert solvent in the presence of the radical polymerization catalyst, the solvent may further contain a nonionic surfactant. The nonionic surfactant is preferably a nonionic surfactant with a polyoxyethylene chain.

The nonionic surfactant with a polyoxyethylene chain is, for example, preferably an ethylene oxide adduct of a polyhydric alcohol fatty acid ester, a block copolymer of hydroxy fatty acid and ethylene oxide, or polyoxyethylene castor oil.

The ethylene oxide adduct of a polyhydric alcohol fatty acid ester is, for example, preferably an ester compound of polyoxyethylene hydrogenated castor oil and polyhydric alcohol fatty acid. The polyhydric alcohol fatty acid is preferably a saturated or unsaturated polyhydric (particularly dihydric) alcohol fatty acid with 14 to 24 carbon atoms (14, 15, 16, 17, 18, 19, 20, 21, 22, 23, or 24). More specifically, the polyhydric alcohol fatty acid is, for example, preferably isopalmitic acid, isostearic acid, or isooleic acid. The average number of moles of added ethylene oxides of polyoxyethylene in the ester compound of polyoxyethylene hydrogenated castor oil and polyhydric alcohol fatty acid is not particularly limited, and can be, for example, about 20 to 100 or about 30 to 70. The ester compound of polyoxyethylene hydrogenated castor oil and polyhydric alcohol fatty acid is particularly preferably polyoxyethylene hydrogenated castor oil isostearate.

The polyoxyethylene castor oil is those with the number of moles of ethylene oxides added being preferably about 2 to 10, and more preferably about 2 to 5.

The block copolymer of hydroxy fatty acid and ethylene oxide can also be rephrased as a copolymer of polyhydroxy fatty acid and polyoxyethylene. The fatty acid of polyhydroxy fatty acid is preferably a fatty acid with about 14 to 22 carbon atoms, preferably myristic acid, palmitic acid, or stearic acid. The hydroxy fatty acid is preferably hydroxymyristic acid, hydroxypalmitic acid, or hydroxystearic acid, and particularly preferably hydroxystearic acid. The hydroxystearic acid is particularly preferably 12-hydroxystearic acid. The polyhydroxy fatty acid is particularly preferably polyhydroxystearic acid. The block copolymer of hydroxy fatty acid and ethylene oxide is particularly preferably a block copolymer of 12-hydroxystearic acid and ethylene oxide.

The nonionic surfactant preferably has an HLB value of 5 to 8.

The nonionic surfactants may be used alone or in a combination of two or more.

The amount of the nonionic surfactant for use is preferably about 1 to 10 parts by mass per 100 parts by mass of the component (I). The upper or lower limit of this range may be, for example, 1.5, 2, 2.5, 3, 3.5, 4, 4.5, 5, 5.5, 6, 6.5, 7, 7.5, 8, 8.5, 9, or 9.5 parts by mass. For example, the range may be 1.5 to 9 parts by mass.

The inert solvent is not particularly limited and is preferably a solvent that dissolves the components (I) to (III) and does not dissolve the obtained crosslinked polymer. Specific examples of such solvents include n-pentane, n-hexane, n-heptane, n-octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, and isobutyl methyl ketone. Of these polymerization solvents, from the standpoint of stable quality and availability, ethylene dichloride, n-hexane, n-heptane, and ethyl acetate are preferable. These polymerization solvents can be used alone or in a combination of two or more.

The amount of the inert solvent for use is not particularly limited and is, for example, preferably 200 to 10000 parts by mass, and more preferably 300 to 2000 parts by mass, per 100 parts by mass of the component (I). The use of the solvent within this range can suitably suppress aggregation of the crosslinked polymer in the progress of the polymerization reaction to enable uniform stirring, and can allow the polymerization reaction to proceed more efficiently.

The atmosphere for performing the polymerization reaction is not particularly limited as long as the polymerization reaction can be performed; the atmosphere is, for example, an inert gas atmosphere such as nitrogen gas or argon gas.

The reaction temperature for the polymerization reaction is not particularly limited as long as the polymerization reaction can be performed; the reaction temperature is, for example, preferably 50 to 90°C, and more preferably 55 to 75°C. Performing a polymerization reaction at these reaction temperatures suitably limits the increase in viscosity of the reaction solution and simplifies reaction control.

The reaction time for the polymerization reaction cannot be generalized because it varies according to the reaction temperature; however, the reaction time is typically 2 to 10 hours.

After completion of the reaction, a crosslinked polymer in the form of fine powder can be obtained, for example, by heating the reaction solution to 80 to 130°C to remove the polymerization solvent.

The crosslinked polymer of the present disclosure can preferably be used as a thickener (in particular, for example, a hydrophilic thickener or gelling agent). The present disclosure preferably also encompasses a thickener comprising the crosslinked polymer of the present disclosure. A viscous composition that is produced by using the crosslinked polymer of the present disclosure as a thickener has, in particular, excellent transparency (i.e., a high degree of transparency) and good skin feel (in particular, excellent spreadability when applied to the skin), and can thus be preferably used, for example, in the preparation of an external composition. The external composition is preferably, for example, an external pharmaceutical composition or a cosmetic product. The cosmetic product is preferably, for example, a skin-care product, a hair composition, or a scalp composition. The content of the crosslinked polymer of the present disclosure in the external composition is not particularly limited and may be, for example, about 0.1 to 2 mass%.

In the present specification, the terms "comprising" and "containing" include the concepts of "consisting essentially of" and "consisting of." The present disclosure also encompasses any and every combination of the elements described in the present specification.

The various properties (characteristics, structures, functions, etc.) described above for each embodiment of the present disclosure may be combined in any way in identifying the subject matter encompassed by the present disclosure. Specifically, the disclosure encompasses all subject matter formed of any and every combination of the combinable properties described in the specification.

### Examples

The following describes embodiments of the present disclosure in more detail with reference to Examples. However, the embodiments of the disclosure are not limited to the Examples below.

### Production of (Meth)acrylic acid/(meth)acrylic Acid Alkyl Ester Crosslinked Copolymer Particles

### Example 1

### Production Method

A 500 mL round separable flask was prepared as a reaction vessel, and 40 g (38 mL) of acrylic acid, 0.40 g of Blemmer VMA-70 (a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate; produced by NOF Corporation) as a (meth)acrylic acid alkyl ester wherein the alkyl has 18 to 24 carbon atoms, and 0.18 g of pentaerythritol tetraallyl ether (PETA) were placed in the flask. Further, 232 g (342 mL) of a solution of 0.16 g of lauroyl peroxide (LPO) as an organic peroxide radical initiator in n-hexane (n-Hex) was added gently. The separable flask containing the solution was fixed to a four-necked separable cover equipped with a stirrer, a thermometer, a glass tube for nitrogen feeding, and a cooling tube. An agitator was equipped with four inclined paddle blades and an anchor-shaped agitator blade, and the inclined paddle blades were a combination of blades with different lengths.

While the solution prepared in the reaction vessel was stirred and mixed uniformly, nitrogen gas was blown into the solution at room temperature to deoxidize the upper space of the reaction vessel and the solution. After sufficient deoxidization, while the internal temperature of the solution was kept at 60 to 65°C, the slurry generated in the reaction was allowed to react in a nitrogen atmosphere for 4 hours with stirring at a peripheral speed of 0.075 to 0.175 m/min. After reacting for 1 hour at 60 to 65°C, a solution of 0.8 g of acrylic acid and 1.6 g of a block copolymer of 12-hydroxystearic acid and polyoxyethylene (Hypermer B246, produced by Croda) as a nonionic surfactant in 1.6 g of n-hexane was added to the reaction vessel.

After the reaction was complete, the generated white slurry was heated to 95 to 105°C to remove n-hexane, and then dried under reduced pressure at 120°C at 4 to 5 mmHg for 8 hours, thereby obtaining 38 g of a white fine powder of a crosslinked copolymer.

### Example 2

### Production Method

The operation of the production method of Example 1 was performed in the same manner, except that the amount of pentaerythritol tetraallyl ether was 0.20 g, thereby obtaining 39 g of a white fine powder of a crosslinked polymer.

### Example 3

### Production Method

The operation of the production method of Example 1 was performed in the same manner, except that the amount of pentaerythritol tetraallyl ether was 0.32 g, that the amount of lauroyl peroxide was 0.10 g, that a mixed solution of 133 g of ethyl acetate and 154 g of cyclohexane (c-Hex) was used instead of n-hexane, and that the reaction time was 8 hours, thereby obtaining 38 g of a white fine powder of a crosslinked polymer.

### Comparative Example 1

### Production Method

The operation of the production method of Example 1 was performed in the same manner, except that the amount of pentaerythritol tetraallyl ether was 0.20 g, and that 0.12 g of dimethyl 2,2'-azobis(isobutyrate) (MAIB) was used instead of lauroyl peroxide, thereby obtaining 38 g of a white fine powder of a crosslinked carboxyl group-containing polymer.

### Comparative Example 2

### Production Method

A 500 mL round separable flask was prepared as a reaction vessel, and 45 g (43 mL) of acrylic acid and 0.25 g of pentaerythritol tetraallyl ether were placed in the flask. Further, 212 g (313 mL) of a solution of 0.10 g of azobisisobutyronitrile (AIBN) in n-hexane was added gently. The separable flask containing the solution was fixed to a four-necked separable cover equipped with a stirrer, a thermometer, a glass tube for nitrogen feeding, and a cooling tube. An agitator was equipped with four inclined paddle blades and an anchor-shaped agitator blade, and the inclined paddle blades were a combination of blades with different lengths.

While the solution prepared in the reaction vessel was stirred and mixed uniformly, nitrogen gas was blown into the solution at room temperature to deoxidize the upper space of the reaction vessel and the solution. After sufficient deoxidization, while the internal temperature of the solution was kept at 55 to 65°C, the slurry generated in the reaction was allowed to react in a nitrogen atmosphere for 4 hours with stirring at a peripheral speed of 0.075 to 0.175 m/min.

After the reaction was complete, the generated white slurry was heated to 95 to 105°C to remove n-hexane, and then dried under reduced pressure at 120°C at 4 to 5 mmHg for 8 hours, thereby obtaining 43 g of a white fine powder of a crosslinked copolymer.

The table below summarizes the conditions for producing the crosslinked polymers of the above Examples.

**Table 1**

| | | (Meth)acrylic acid | Ratio of monomer fed to reaction vessel (parts by volume) | (Meth)acrylic acid alkyl ester | Ratio relative to acrylic acid (parts by mass) | Compound with two or more ethylenically unsaturated groups | Ratio relative to acrylic acid (parts by mass) | Radical polymerization catalyst | Ratio relative to acrylic acid (parts by weight) | Nonionic surfactant | Ratio relative to acrylic acid (parts by weight) | Inert solvent | Reaction temperature (°C) | Reaction time (hours) |
|---|---|---|---|---|---|---|---|---|---|---|---|---|---|---|
| Example | 1 | Acrylic acid | 10 | Blemmer VMA-70 | 1 | PETA | 0.45 | LPO | 0.40 | Hypermer B246 | 4 | n-Hex | 60-65 | 4 |
| | 2 | Acrylic acid | 10 | Blemmer VMA-70 | 1 | PETA | 0.50 | LPO | 0.40 | Hypermer B246 | 4 | n-Hex | 60-65 | 4 |
| | 3 | Acrylic acid | 10 | Blemmer VMA-70 | 1 | PETA | 0.80 | LPO | 0.25 | Hypermer B246 | 4 | AcOEt:c-Hex=50:50 | 60-65 | 8 |
| Comparative Example | 1 | Acrylic acid | 10 | Blemmer VMA-70 | 1 | PETA | 0.50 | MAIB | 0.30 | Hypermer B246 | 4 | n-Hex | 60-65 | 4 |
| | 2 | Acrylic acid | 12 | - | - | PETA | 0.56 | AIBN | 0.22 | - | - | n-Hex | 55-65 | 4 |

### Evaluation of Physical Properties of (Meth)acrylic acid/(meth)acrylic Acid Alkyl Ester Crosslinked Copolymer Particles

### Measurement with Ubbelohde Dilution Viscometer

A 0.015 mass% neutralized aqueous solution of the obtained crosslinked polymer particles was prepared. More specifically, 288.8 g of ion-exchanged water was placed in a 500 mL beaker, and 1.5 g of crosslinked polymer particles were gradually added with stirring at 1500 rpm with a dispersion mixer. After subsequent stirring for 30 minutes, the dispersion mixer was turned off, and 9.7 g of a 6 mass% aqueous sodium hydroxide solution was added, followed by stirring the mixture for 30 seconds with a handheld mixer, thereby obtaining a 0.5 mass% neutralized aqueous solution of crosslinked carboxyl group-containing polymer particles (pH 7; measured with a pH meter at 25°C).

The neutralized aqueous solution was then further diluted with ion-exchanged water to 0.015 mass%. The 0.015 mass% neutralized aqueous solution was measured for flow time at 25.0 ± 0.01°C with an Ubbelohde dilution viscometer. The Ubbelohde dilution viscometer for use was one with a flow time of 100 to 200 seconds during solvent measurement in accordance with JIS K-7367-1 (Asahi Glassplant, Inc. 2B (Cat. No. 4804-09)). The measurement method was performed in accordance with JIS K-7367-1. More specifically, the flow time of the 0.015 mass% neutralized aqueous solution was first measured, and then a predetermined amount of a solvent (water) was further added for dilution, followed by measuring the flow time. This operation was performed several times (about 4 to 5 times) to measure the flow time at each concentration. With flow time (t) at each concentration and solvent-only flow time (t₀), η_{gp} can be determined according to the following relational expression. ηₛₚ is a specific viscosity (indicating the rate of increase in viscosity caused by a solute in a solvent).
*η*ₛₚ = (*η*-*η*₀)/*η*₀ = (*ρ*t-*ρ*₀t₀)/*ρ*₀t₀ ≈ (t-t₀)/t₀ (*ρ*₀: solvent viscosity, t₀: solvent flow time, *ρ*: solution viscosity, t: solution flow time)

Then, the results are plotted with concentration (c) on the horizontal axis and (ηₛₚ/c) on the vertical axis. Martin's equation ηₛₚ/c = [η]e^{k[η]c} with exponential approximation gives [η] and k. (Plotting the measured values gives exponential approximation in the Martin's equation. The intercept of the extrapolation of zero concentration is intrinsic viscosity [η].) [η] denotes an intrinsic viscosity, k denotes an interaction coefficient, and k[η] denotes the product of these. The unit for [η] is dl/g.

### Yield Value Measurement

The viscosity of the 0.5 mass% neutralized aqueous solution of the crosslinked polymer particles prepared as described above was measured with a dynamic viscoelasticity analyzer (rheometer: AR-2000ex from TA Instruments Japan, Inc.). The steady-state flow viscosity at a shear stress of 1.0 × 10⁻⁵ to 1.0 × 10³ (Pa) was measured by using a 60 mm (diameter), 4-degree cone with the temperature set to 25°C under steady-state conditions that satisfied the following items: percentage tolerance 1.0, consecutive within tolerance 3, and maximum point time 10 min. The point at which the strain changed abruptly with respect to shear stress was determined to be the yield value (yield stress, Pa).

### Elastic Modulus Measurement

Frequency sweep measurement was performed in accordance with the following procedure with respect to the 0.5 mass% neutralized aqueous solution of crosslinked polymer particles prepared as described above to determine the G' value (storage elastic modulus) at 25°C at an angular frequency of 0.1 rad/s.

### Frequency Sweep Measurement Conditions

Rheometer: TA Instruments (model number: AR 2000ex)
Plate: 4°, 60 mm (diameter)
Measurement temperature: 25°C
Angular frequency: 0.1 to 300 rad/s
Strain: 1%

### Production of Liquid Composition Using (Meth)acrylic acid/(meth)acrylic Acid Alkyl Ester Crosslinked Copolymer Particles

A liquid composition (which is useful, for example, as a hair gel composition) was produced according to the formulation in the table below. Specifically, materials 1 and 2 were mixed at room temperature with a dispersion mixer (5000 rpm, 30 minutes), and material 3 was then added and stirred until uniform. Material 4 was then added and stirred until uniform. The GAFQUAT 755N used as material 4 was a 20 mass% aqueous solution of polyquaternium-11 (weight average molecular weight: about 1 million).

**Table 2**

| Material | INCI Name | Product name | Wt% |
|---|---|---|---|
| 1 | Purified water | | Residue |
| 2 | (Acrylates/C10-30 alkyl acrylate) crosspolymer | Crosslinked polymer of Examples 1 to 3 and Comparative Examples 1 and 2 were used | 0.5 |
| 3 | 50% Aminomethyl propanol | 2-Amino-2-methyl-1 -propanol | 1.5 |
| 4 | Polyquaternium-11 | GAFQUAT 755N | 5.0 |

The transparency and tactile sensations of the liquid compositions prepared by using the crosslinked polymers obtained in the Examples and Comparative Examples were evaluated as described below.

### Transparency

The transparency of the liquid compositions was measured with a UV-visible spectrophotometer (UV-1850 from Shimadzu Corporation). Specifically, each of the liquid compositions was filled in a UV measurement cell made of PMMA (polymethyl methacrylate) and defoamed with a centrifuge (2000 rpm, 5 minutes) to measure the transmittance (%) by UV measurement at a wavelength of 425 nm. The results were then evaluated according to the following evaluation criteria A.

### Evaluation Criteria

I: 70% or more
II: 50% to 69%
III: less than 50%

### Tactile Sensation

A total of ten panelists, including men and women, was selected as evaluators. Each of the liquid compositions was applied and spread over the back of their hands, and the number of panelists who felt that the composition spread well was recorded. Then, evaluation was performed according to the following evaluation criteria.

### Evaluation Criteria

I: 9 or more panelists
II: 8 to 6 panelists
III: 5 or less panelists

The following table summarizes the results of the evaluation of the physical properties of the crosslinked polymers described above and the results of the evaluation of the physical properties of the liquid compositions described above.

**Table 3**

| | Rheometer | | Ubbelohde Viscometer | | | Evaluation of liquid composition | | | |
|---|---|---|---|---|---|---|---|---|---|
| | Yield stress [Pa] | Storage elastic modulus [Pa] | [η] | k | k[η] | Transparency | | Spreadability | |
| Ex. 1 | 22.4 | 372 | 46.9 | 1.23 | 57.6 | 71.2% | I | 9 panelists | I |
| Ex. 2 | 23.4 | 292 | 45.7 | 1.60 | 73.1 | 75.1% | I | 9 panelists | I |
| Ex. 3 | 21.1 | 220 | 51.2 | 2.21 | 113.0 | 77.4% | I | 10 panelists | I |
| Comp. Ex. 1 | 17.6 | 388 | 44.2 | 1.15 | 50.7 | 39.9% | III | 8 panelists | II |
| Comp. Ex. 2 | 21.1 | 676 | 28.6 | 1.34 | 38.2 | 26.8% | III | 5 panelists | III |

## Claims

1. A (meth)acrylic acid/(meth)acrylic acid alkyl ester crosslinked copolymer obtained by polymerizing
(meth)acrylic acid,
a (meth)acrylic acid alkyl ester wherein the alkyl has 18 to 24 carbon atoms, and
a compound with two or more ethylenically unsaturated groups,
wherein
a 0.5 mass% neutralized aqueous solution of the crosslinked copolymer at 25°C has a yield stress of 20 Pa or more, and
a 0.5 mass% neutralized aqueous solution of the crosslinked copolymer at 25°C has a storage elastic modulus (G') of 100 to 400 Pa at an angular frequency of 0.1 rad/s.

2. The crosslinked copolymer according to claim 1,
wherein k is 1.2 to 6.0 wherein k represents an interaction coefficient as determined by measuring a neutralized aqueous solution of the crosslinked copolymer having an initial concentration of 0.015 mass% with an Ubbelohde dilution viscometer.

3. The crosslinked copolymer according to claim 1 or 2,
wherein the (meth)acrylic acid alkyl ester wherein the alkyl has 18 to 24 carbon atoms is
at least one member selected from the group consisting of eicosanyl (meth)acrylate, behenyl (meth)acrylate, and tetracosanyl (meth)acrylate, or
a mixture of two to four members selected from the group consisting of stearyl (meth)acrylate, eicosanyl (meth)acrylate, behenyl (meth)acrylate, and tetracosanyl (meth)acrylate.

4. The crosslinked copolymer according to claim 1 or 2,
wherein the compound with two or more ethylenically unsaturated groups is at least one member selected from the group consisting of sucrose allyl ether and pentaerythritol allyl ether.

5. The crosslinked copolymer according to claim 1 or 2, which is a polymer comprising 0.5 to 3 parts by mass of the (meth)acrylic acid alkyl ester wherein the alkyl has 18 to 24 carbon atoms per 100 parts by mass of the (meth)acrylic acid.

6. The crosslinked copolymer according to claim 1 or 2, which is a polymer comprising 0.15 to 2 parts by mass of the compound with two or more ethylenically unsaturated groups per 100 parts by mass of the (meth)acrylic acid.

7. A thickener comprising the crosslinked copolymer of claim 1 or 2.

8. An external composition comprising the crosslinked copolymer of claim 1 or 2.

9. A method for producing a (meth)acrylic acid/(meth)acrylic acid alkyl ester crosslinked copolymer crosslinked by a compound with two or more ethylenically unsaturated groups,
the method comprising
reacting (meth)acrylic acid, a (meth)acrylic acid alkyl ester wherein the alkyl has 18 to 24 carbon atoms, and a compound with two or more ethylenically unsaturated groups in an inert solvent in the presence of a radical polymerization catalyst,
wherein the radical polymerization catalyst is an organic peroxide and is present in an amount of 0.1 to 1 part by mass per 100 parts by mass of the (meth)acrylic acid.
